# EUROPEAN PATENT SPECIFICATION

(11) **EP 0 543 633 B1**
(45) Date of publication and mention of the grant of the patent: **30.10.1996**
(21) Application number: 92310511.8
(22) Date of filing: 18.11.1992
(51) Int. Cl.: C07C 209/10

(54) **Process for producing 4-amino-3-fluorobenzotrifluoride**
Verfahren zur Herstellung von 4-Amino-3-Fluorobenzotrifluorid
Procédé de préparation de 4-amino-3-fluorobenzotrifluoride

(30) Priority: 20.11.1991 JP 304608/91
(43) Date of publication of application: 26.05.1993
(73) Proprietor: SUMITOMO CHEMICAL COMPANY LIMITED, Osaka-shi, Osaka 541 (JP)
(72) Inventor: Sakamoto, Noriyasu, Nishinomiya-shi, Hyogo-ken (JP); Taki, Toshiaki, Toyonaka-Shi, Osaka-fu (JP); Matsuo, Noritada, Itami-shi, Hyogo-ken (JP)
(74) Representative: Cresswell, Thomas Anthony

(56) References cited:
- EP-A- 0 315 863
- EP-A- 0 381 010

## Description

This invention relates to an industrially advantageous process for producing 4-amino-3-fluorobenzo-trifluoride, which is useful as an intermediate for the production of insecticides.

EP-A-0315863 describes a process for the preparation of fluorine- and chlorine-containing trifluoromethylaminobenzenes. The process comprises treating a starting compound bearing two fluorine substituents and at least one further substituent selected from fluorine and chlorine with ammonia. The starting compound is accordingly substituted by three electronegative groups, which facilitates attack by the nucleophilic ammonia.

The processes known for producing 4-amino-3-fluorobenzotrifluoride, as described in J. Org. Chem., 50, 4576 (1985) and EP-A-0246061, may be illustrated as follows:

J. Org. Chem., 50, 4576 (1985)

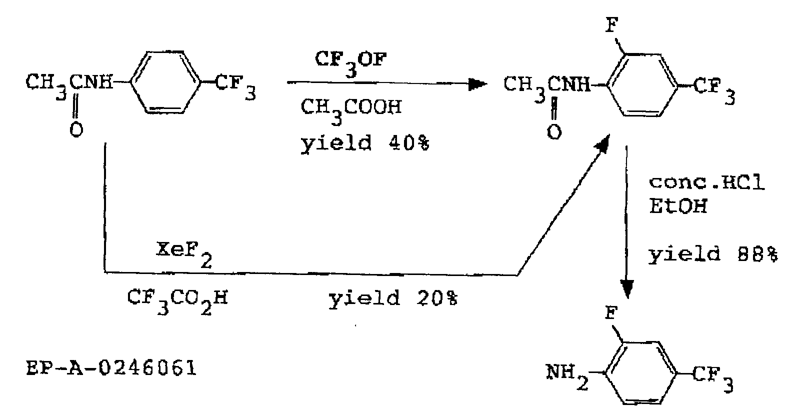

However, these processes are not necessarily satisfactory for the production of 4-amino-3-fluorobenzotrifluoride on a commercial scale. That is to say, trifluoromethyl hypofluorite is not readily available because of the need for special fluorine-handling techniques, while xenon difluoride is an expensive reagent. Furthermore, fluorination with XₑF₂ is not very regioselective, which means that the purity of the product is not good. Also, each of these processes involves two reaction steps inclusive of hydrolysis (deacetylation) and cannot therefore be said to be commercially advantageous.

Against this background, it was sought to develop a process for producing 4-amino-3-fluorobenzotrifluoride which is much improved from an industrial point of view. Accordingly, the present invention provides a process for producing 4-amino-3-fluorobenzotrifluoride which comprises reacting 3,4-difluorobenzotrifluoride with anhydrous ammonia under pressure, wherein either no solvent is used or the solvent consists of liquid ammonia.

The anhydrous ammonia which is used may be either liquid ammonia or gaseous ammonia. In the process of the invention, the 3,4-difluorobenzotrifluoride and anhydrous ammonia may be placed into a reactor in any order. Thus, the two reactants may simultaneously be charged into the reactor and then the reaction be conducted under the reaction conditions described below. Alternatively either of the two reactants may be introduced into the reactor first and then, under the following reaction conditions, the other reactant may be charged into the reactor in portions to effect the reaction gradually.

The anhydrous ammonia is generally used in an amount of about 1 to about 100 moles, preferably (from an economic point of view) about 1 to about 20 moles, per mole of 3,4-difluorobenzotrifluoride. The reaction is typically carried out at a temperature in the range of about 30 to about 300°C, preferably about 80 to about 120°C, usually for a period of about 1 to about 100 hours. The reaction is preferably conducted in a closed, pressure-resistant vessel, such as an autoclave. In this case the reaction is allowed to proceed under a pressure resulting from the reaction mixture. The reaction is generally conducted at a pressure ranging usually from slightly above atmospheric pressure, for example about 1.03 kg/cm², to about 100 kg/cm². Preferably the pressure range is 20kg/cm² to about 100 kg/cm², although the pressure may vary depending on the reactor capacity and reaction temperature.

In order to isolate the desired 4-amino-3-fluorobenzotrifluoride, unreacted 3,4-difluorobenzotrifluoride may be recovered from the reaction mixture. However, if the unreacted 3,4-difluorobenzotrifluoride is present in the reaction mixture only in a small amount, such a recovery step may be omitted. The desired 4-amino-3-fluorobenzotrifluoride and unreacted 3,4-difluorobenzotrifluoride can be recovered and separated from the reaction mixture, for example, by distillation or by organic solvent extraction and subsequent distillation. If the reaction mentioned above is carried out in a reactor equipped with a distillation apparatus, the desired 4-amino-3-fluorobenzotrifluoride, and unreacted 3,4-difluorobenzotrifluoride can be directly recovered from the reaction mixture and separated from each other by distillation of the reaction mixture. Alternatively, the reaction mixture may be subjected to an organic solvent extraction after the addition thereto of water and a low boiling organic solvent, such as diethyl ether, dichloromethane, n-hexane, etc. After separation of the aqueous layer, the organic layer is distilled, whereby the desired product 4-amino-3-fluorobenzotrifluoride and unreacted 3,4-difluorobenzotrifluoride can be recovered and separated from each other. When necessary, the product 4-amino-3-fluorobenzotrifluoride can be further purified by chromatography or distillation, for instance.

The unreacted 3,4-difluorobenzotrifluoride thus recovered can be recycled as a starting material of the present prepartion process.

The desired 4-amino-3-fluorobenzotrifluoride may be converted to benzoylurea insecticides by the method described, for example, in EP-A-0246061.

The starting material for carrying out the process of the present invention, namely 3,4-difluorobenzotrifluoride, can be prepared by a known method, for instance, by the method described in US-A-4 937 396.

The following working examples further illustrate the present invention.

### Example 1

A stainless steel autoclave was charged with 10.0 g (0.055 mole) of 3,4-difluorobenzotrifluoride and then with 9.34 g (0.55 mole) of anhydrous ammonia. The charge was stirred at an autoclave temperature of 100-110°C for 38 hours, the pressure being 50 kg/cm² at its maximum. The autoclave was then cooled to 20°C and the pressure in the autoclave was allowed to return to ordinary (atmospheric) pressure.

The reaction mixture was subjected to distillation at ordinary (atmospheric) pressure to give 7.7 g of 3,4-difluorobenzotrifluoride boiling at 103-105°C. The residue was then purified by silica gel column chromatography (eluent: n-hexane/ethyl acetate = 4/1) to give 1.97 g of 4-amino-3-fluorobenzotrifluoride.
Yield 20% (corrected yield (see note ) 87%);
n$\frac{\text{22.4}}{\text{D}}$ = 1.4642.
(Note)$\text{Corrected yield =} \frac{{\text{A}}_{\text{MW}} \text{x B}}{{\text{B}}_{\text{MW}} {\text{x (A}}_{\text{0}} {\text{- A}}_{\text{1}} \text{)}} \text{x 100 (%)}$ where
- A_{MW} =: Molecular weight of 3,4-difluorobenzotrifluoride,
- B_{MW} =: Molecular weight of 4-amino-3-fluorobenzotrifluoride,
- B =: Weight (g) of 4-amino-3-fluorobenzotrifluoride,
- A₀ =: Weight (g) of 3,4-difluorobenzotrifluoride charged,
- A₁ =: Weight (g) of 3,4-difluorobenzotrifluoride recovered.

### Example 2

A stainless steel autoclave was charged with 20.0 g (0.11 mole) of 3,4-difluorobenzotrifluoride and then with 9.35 g (0.55 mole) of anhydrous ammonia. The charge was stirred at an autoclave temperature of 100-120°C for 40 hours, the pressure being 60 kg/cm² at its maximum. The autoclave was then cooled to 20°C and the pressure in the autoclave was allowed to return to ordinary (atmospheric) pressure.

The reaction mixture was subjected to distillation at ordinary (atmospheric) pressure to give 15.8 g of 3,4-difluorobenzotrifluoride boiling at 103-105°C. The residue was then subjected to distillation under reduced pressure to give 3.76 g of 4-amino-3-fluorobenzotrifluoride (b.p. 109-111°C/25 mmHg). Yield 19% (corrected yield (see note) 91%).

(Note) Same as mentioned above.

As a process for producing 4-amino-3-fluorobenzotrifluoride, the process of the invention is advantageous over the prior art processes from the industrial standpoint since the desired product can be obtained with high selectivity in one step by reacting 3,4-difluorobenzotrifluoride with ammonia, which is inexpensive, under pressure and since unreacted 3,4-difluorobenzotrifluoride can be recovered from the same reactor and can readily be recycled.

## Claims

1. A process for producing 4-amino-3-fluorobenzotrifluoride which comprises reacting 3,4-difluorobenzotrifluoride with anhydrous ammonia under pressure, wherein either no solvent is used or the solvent consists of liquid ammonia.

2. A process as claimed in claim 1 wherein the reaction is carried out at a pressure of 1.03 to 100 kg/cm².

3. A process as claimed in claim 2 wherein the reaction is carried out at a pressure of 20 to 100 kg/cm².

4. A process as claimed in any one of claims 1 to 3 wherein 1 to 100 moles of anhydrous ammonia is used per mole of 3,4-difluorobenzotrifluoride.

5. A process as claimed in any one of claims 1 to 4 wherein the reaction is carried out at a temperature of 30 to 300°C.

6. A process as claimed in claim 5, wherein the reaction is carried out at a temperature of 80 to 120°C.

7. A process according to any one of claims 1 to 6 comprising recovering and separating 4-amino-3-fluorobenzo trifluoride from the reaction mixture.

8. A process according to any one of claims 1 to 6 comprising recovering and separating 4-amino-3-fluorobenzotrifluoride and unreacted 3,4-difluorobenzotrifluoride from the reaction mixture.

9. A process as claimed in claim 7 or claim 8 wherein the unreacted 3,4-difluorobenzotrifluoride is recovered and separated by distillation.

## Patentansprüche

1. Verfahren zur Herstellung von 4-Amino-3-fluorbenzotrifluorid , welches umfaßt: Umsetzen von 3,4-Difluorbenzotrifluorid mit wasserfreiem Ammoniak unter Druck, wobei entweder kein Lösungsmittel verwendet wird oder das Lösungsmittel aus flüssigem Ammoniak besteht.

2. Verfahren nach Anspruch 1, wobei die Umsetzung bei einem Druck von 1,03 bis 100 kg/cm² erfolgt.

3. Verfahren nach Anspruch 2, wobei die Umsetzung bei einem Druck von 20 bis 100 kg/cm² erfolgt.

4. Verfahren nach einem der Ansprüche 1 bis 3, wobei 1 bis 100 Mol wasserfreier Ammoniak pro Mol 3,4-Difluorbenzotrifluorid verwendet wird.

5. Verfahren nach einem der Ansprüche 1 bis 4, wobei die Umsetzung bei einer Temperatur von 30 bis 300° C erfolgt.

6. Verfahren nach Anspruch 5, wobei die Umsetzung bei einer Temperatur von 80 bis 120° C erfolgt.

7. Verfahren nach einem der Ansprüche 1-6, welches die Gewinnung und Abtrennung von 4-Amino-3-fluorbenzotrifluorid aus der Reaktionsmischung umfaßt.

8. Verfahren nach einem der Ansprüche 1 bis 6, welches die Gewinnung und Abtrennung von 4-Amino-3-fluorbenzotrifluorid und nicht umgesetztem 3,4-Difluorbenzotrifluorid aus der Reaktionsmischung umfaßt.

9. Verfahren nach Anspruch 7 oder Anspruch 8, wobei das nicht umgesetzte 3,4-Difluorbenzotrifluorid durch Destillation gewonnen und abgetrennt wird.

## Revendications

1. Procédé de production de 4-amino-3-fluorobenzotrifluorure qui comprend la réaction du 3,4-difluorobenzotrifluorure avec l'ammoniac anhydre sous pression, et dans lequel, soit il n'est pas utilisé de solvant, soit le solvant est l'ammoniac liquide.

2. Procédé selon la revendication 1, dans lequel la réaction est effectuée à une pression comprise entre 1,03 et 100 kg/cm².

3. Procédé selon la revendication 1, dans lequel la réaction est effectuée à une pression comprise entre 20 et 100 kg/cm².

4. Procédé selon l'une quelconque des revendications 1 à 3, dans lequel 1 à 100 moles d'ammoniac anhydre sont utilisées par mole de 3,4-difluorobenzotrifluorure.

5. Procédé selon l'une quelconque des revendications 1 à 4, dans lequel la réaction est effectuée à une température comprise entre 30 et 300°C.

6. Procédé selon la revendication 5, dans lequel la réaction est effectuée à une température comprise entre 80 et 120°C.

7. Procédé selon l'une quelconque des revendications 1 à 6, comprenant la récupération, et la séparation du 4-amino-3-fluorobenzotrifluorure, à partir du mélange réactionnel.

8. Procédé selon l'une quelconque des revendications 1 à 6, comprenant la récupération, et la séparation du 4-amino-3-fluorobenzotrifluorure, et du 3,4-difluorobenzotrifluorure non transformé, à partir du mélange réactionnel.

9. Procédé selon la revendication 7, ou la revendication 8, dans lequel le 3,4-difluorobenzotrifluorure non transformé est récupéré et séparé par distillation.
